(19) Europäisches Patentamt European Patent Office Office européen des brevets

(11) EP 2 387 893 B1

## (12) EUROPEAN PATENT SPECIFICATION

(45) Date of publication and mention
of the grant of the patent:
**18.06.2014 Bulletin 2014/25**

(21) Application number: **10731346.2**

(22) Date of filing: **14.01.2010**

(51) Int Cl.:
*A41D 1/08* (2006.01)     *A41D 7/00* (2006.01)
*A41D 13/00* (2006.01)     *A61F 13/08* (2006.01)

(86) International application number:
**PCT/JP2010/050657**

(87) International publication number:
**WO 2010/082677 (22.07.2010 Gazette 2010/29)**

(54) **MEDICAL CLOTHING AND SPORTS WEAR**

MEDIZINISCHE KLEIDUNG UND SPORTKLEIDUNG

VÊTEMENT À USAGE MÉDICAL ET SPORTIF

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **15.01.2009 JP 2009007037**

(43) Date of publication of application:
**23.11.2011 Bulletin 2011/47**

(73) Proprietors:
• **Goldwin Inc.**
**Toyama 932-0112 (JP)**
• **Goldwin Technical Center Inc.**
**Oyabe-shi, Toyama 932-0193 (JP)**

(72) Inventors:
• **KANADA, Tsuguhiro**
**Oyabe-shi**
**Toyama 932-0193 (JP)**
• **ITO, Ryota**
**Oyabe-shi**
**Toyama 932-0193 (JP)**
• **ARAI, Gen**
**Tokyo 150-8517 (JP)**

(74) Representative: **Foot, Paul Matthew James**
**Withers & Rogers LLP**
**4 More London Riverside**
**London**
**SE1 2AU (GB)**

(56) References cited:
JP-A- 10 110 306      JP-A- 10 130 915
JP-A- 10 130 915      JP-A- 2001 172 805
JP-A- 2002 105 712    JP-A- 2008 007 863
JP-U- 3 016 025       JP-U- 3 024 047

**Description**

Technical Field

The present invention relates to medical or sports wear. More specifically, the invention relates to medical or sports wear having graded wear pressure, and having cuttings for a high follow property during exercise activity.

Background Art

Conventional tights are known which have a structure that applies stepwise pressure to the ankles, calves and femoral regions in order to promote venous return (PTL 1 and PTL 2).

However, such tights are associated with problems of durability and elasticity when applied as sports wear. Furthermore, the tights do not have a specific pattern design with a follow-up property during exercise activity for the knee section, which undergoes the greatest degree of extension in the human body, and therefore problems occur such as changes in wear pressure, stretched feeling and movement impedance during times of motion.

For sports wear as well, graded wear pressure has been proposed to reduce fatigue on the legs or improve performance during sports, or to promote fatigue recovery after sports (PTL 3 and PTL 4).

In PTL 3, for example, there are disclosed long tights made of an elastic material, wherein wear pressure on the femoral region covering the quadriceps femoris muscle, femoral flexor group and adductor muscle group and wear pressure on the crural extensor group and the lower leg covering the crural extensor group and crural flexor group differ on a border line running directly under the knee joint, with wear pressure on the lower leg being higher than the wear pressure on the femoral region.

However, these long tights are not cut with a high follow property for exercise activity, and therefore excess fabric results behind the knee during exercise, creating problems such as an uncomfortable feel and impedance of movement of the lower extremities.

Furthermore, with the long tights described in PTL 3 it is difficult to apply the desired wear pressure to the ankles.

Since most tights have a construction that reaches from the feet to the femoral region or waist (PTL 1 and PTL 2), the desired wear pressure can be applied to the ankles. On the other hand, a problem occurs when wear pressure is increased on ankles in sports wear, which usually has a construction from the ankles to the waist and not on the feet, as it becomes particularly difficult to remove the wear from the ankles.

In PTL 4, there is disclosed compressive wear for covering of a body part with a muscle ridge, the wear comprising a first elastic material panel and a second elastic material panel joined thereto with a seam, wherein at least a portion of the seam corresponds to at least a portion of the muscle ridge.

However, although the compressive wear employs an elastic material, it has numerous crossing and stitched sections that limit the expansion and contraction of the material, and therefore exercise activity tends to be inhibited.

Furthermore, with the compressive wear described in PTL 4, it is difficult to apply the desired wear pressure to the ankles, for the same reason mentioned above.

In PTL5 a sports garment comprising an elastic material is disclosed having a front part, a back part and inner and outer parts located between the front part and the back part. Knee supporting parts are provided on the inner and outer sides of the knee.

A demand therefore exists for sports wear that reduces the extra fabric on the popliteal spaces by being cut in a manner for a high follow property for sports activity, and has minimal unpleasant sense of pressure and a comfortable feel during wear. In addition, conventional sports wear does not have a design that applies desired wear pressure to the ankles, and a demand therefore exists for medical and sports wear that can promote venous return by decremental wear pressure from the ankle to the femoral region, for example. The same demand exists for medical wear as well.

Citation List

Patent Literature

PTL 1 Japanese Unexamined Patent Publication No. 2000-290806
PTL 2 Japanese Unexamined Patent Publication HEI No. 3-279403
PTL 3 Japanese Unexamined Patent Publication HEI No. 10-130915
PTL 4 Japanese Patent Public Inspection No. 2008-513623
PTL 5 Japanese Unexamined Patent Publication HEI No. 10 - 110306

Summary of Invention

Technical Problem

It is an object of the present invention to provide medical and sports wear that facilitates knee bending during sports, minimizes excess fabric on the popliteal spaces, has low unpleasant sense of pressure and a comfortable feeling during wear. It is another object of the invention to provide medical and sports wear that promotes venous return, reduces fatigue on the legs during sports, improves sports ability, reduces fatigue after sports and promotes rapid fatigue recovery.

Solution to Problem

As a result of much diligent research directed toward solving the aforementioned problems of the prior art, the present inventors have found that such problems can be overcome by medical or sports wear comprising an elastic material wherein the medical or sports wear has a crescent-shaped changing part on the inner and outer sides of the knee, and graded wear pressure is applied to the leg during wear, and the invention has been completed upon this finding.
Specifically, the present invention relates to a medical or sports wear according to claim 1. Various embodiments are covered by the dependent claims.

Advantageous Effects of Invention

The medical and sports wear of the invention, which is cut with a high follow property for sports activity, facilitates knee bending during sports, minimizes excess fabric on the popliteal spaces, has low unpleasant sense of pressure and a comfortable feeling during wear.
In addition, the medical and sports wear of the invention, which applies graded wear pressure to the leg and especially decremental wear pressure from the ankle to the femoral region, therefore promotes venous return, reduces fatigue on the legs during sports, improves sports ability, reduces fatigue after sports and promotes rapid fatigue recovery.

Brief Description of Drawing

Fig. 1 is an outer side view of the left foot portion of sports tights as one embodiment of the medical or sports wear of the invention.
Fig. 2 is an inner side view of the left foot portion of sports tights as one embodiment of the medical or sports wear of the invention. The right foot section will not be explained.
Fig. 3 shows the crescent-shaped changing part on the outer side.
Fig. 4 is a front view and rear view of sports tights as one embodiment of the medical or sports wear of the invention.
Fig. 5 is a left side view showing an image of walking with sports tights as one embodiment of the medical or sports wear of the invention.
Fig. 6 is an illustration of a wooden last that can be used to measure wear pressure for medical or sports wear of the invention.
Fig. 7 is an illustration of an example of a variation on a crescent-shaped changing part.
Fig. 8 is an illustration of a crescent-shaped changing part used for the invention.

Description of Embodiments

Preferred embodiments of the invention will now be explained with reference to the accompanying drawings. It is to be understood, however, that the invention is not restricted by the specific embodiments described herein.

[Medical or sports wear]

[Medical wear]

As used herein, "medical wear" refers to clothing that qualifies as Class I General Medical Equipment, among medical equipment established by the Pharmaceutical Affairs Law. There are no particular restrictions on the medical wear of the invention, and examples include pantyhose, spats and tights.
The medical wear of the invention applies graded wear pressure to the legs, promoting venous return in the lower extremities and preventing swelling, and is therefore suitable for standing work, desk work and movement in airplanes or electric trains.

[Sports wear]

As used herein, "sports" is a general term for physical exercise including the elements of recreation, competition and body training, and examples thereof include not only track, baseball, softball, tennis, handball, gymnastics, table tennis, badminton, soccer, basketball, volleyball, bicycling, skiing, snowboarding, skating, swimming and boat racing, but also endurance sports, running, walking, trekking, mountaineering and golfing.

Examples of sports wear of the invention include sports tights, spats, swimming wear, tights and body suits.

The medical wear or sports wear of the invention can apply desired wear pressure to the ankles in a form without a foot section, but the wear of the invention is not limited to such a form, and it may be wear with a foot section, such as pantyhose or tights with ankle sections.

[Elastic material]

The medical or sports wear of the invention comprises an elastic material.

As used herein, the material is an elastic warp knit obtained by knitting from elastic yarn and non-elastic yarn. The elastic yarn is preferably polyurethane elastic yarn, polyether/ester elastic yarn or polyamide elastic yarn, or a rubber-based yarn made of natural rubber or synthetic rubber or semisynthetic rubber, or elastic yarn obtained by compositing or mixing these with organic synthesis resins, and the yarn itself preferably has rubber-like elasticity.

The elastic yarn may be a monofilament or multifilament yarn. The elastic yarn may also be gray yarn, or finished yarn covered with non-elastic yarn or elastic yarn. The elastic yarn is most preferably polyurethane elastic yarn. The size of the elastic yarn is preferably 44 decitex (dtex)-78 decitex.

The non-elastic yarn may be a filament yarn or spun yarn. Specifically, filament yarns include combined fibers of rayon, acetate, polyamide, polyester, acryl, polypropylene or vinyl chloride, and silk (silk yarn), textured yarn, false-twisted finished yarn or colored yarn, or composites of these yarns. These are preferred as they are easily doubled, stable yarns. The spun yarn is staple composed of combined fibers of natural fibers such as (tree) cotton, wool, hemp or silk, or rayon, polyamide, polyester, acrylonitrile, polypropylene or vinyl chloride, and these may be simple or mix spun yarn. The filament yarn is preferably in the range of 22-78 decitex and more preferably in the range of 22-55 decitex.

Examples of such materials include elastic warp knits comprising nylon as reed L1 and polyurethane elastic fiber as reed L2 using both in full set to form a denbigh texture with L1=1023, L2=1210, with knitting to a polyurethane mixing ratio of 20-40% using a common tricot knitting machine.

The elastic material used for the invention preferably has an elongation percentage in the circumferential length direction that is greater than the elongation percentage in the height direction of the material.

As used herein, the term "height direction" means the direction of the height of the wearer, while the term "circumferential length direction" means the direction perpendicular to the height direction.

The term "elongation percentage" means the percentage of the difference between the maximum elongation length (A) that maintains a linear relationship between load and elongation, and the original length (B), with respect to the original length.

The elongation percentage can be expressed by the following formula.

$$\texttt{Elongation percentage (\%) = 100} \times \texttt{(A-B)/B}$$

The elongation percentage can be measured by the following method. A 5 cm-wide, 15 cm-long test piece is set in a Tensilon constant-extension tensile tester, the jig is set to a spacing of 5 cm, and testing is conducted at a pull rate of 5 cm/min until the test piece undergoes fracture. After the test, a graph is drawn with elongation on the abscissa and load on the ordinate, and the elongation percentage is calculated from the maximum elongation length at the point at which load and elongation deviate from a linear relationship. The Tensilon constant-extension tensile tester may be, for example, a RTC-1210A by Orientech Co., Ltd.

If the elongation percentage in the circumferential length direction of the material is greater than in the height direction of the material, especially at the ankles, it is possible to apply a constant degree of wear pressure during wear while maintaining removability.

The elongation percentage in the circumferential length direction of the material used for the invention is preferably 150%-500% and more preferably 150%-300%, from the viewpoint of ensuring removability at the ankle section, ensuring enlargement to a suitable size and ensuring the desired wear pressure on the ankles.

Because the material used for the invention has a high elongation percentage, there is little variation in wear pressure even with enlarged muscles or significant curvature of the foot.

[Crescent-shaped changing part]

The medical or sports wear of the invention has a crescent-shaped changing part on the inner and outer sides of the knee. According to one embodiment of the invention, the crescent-shaped changing part comprises a long arc x close to a kneecap and a short arc y close to a popliteal space, wherein the long arc x close to a kneecap and short arc y close to a popliteal space are each connected to the front body and back body, producing a dimensional difference between the front body and back body. Also, the dimensional difference allows the medical or sports wear of the invention to follow bending of the human skin, and especially the knee.

The crescent-shaped changing part of the inner and outer sides of the knee may be symmetrical or asymmetrical, and for example, the crescent-shaped changing part on the inner side of the knee can be smaller than the crescent-shaped changing part on the outer side of the knee.

In medical or sports wear without such a crescent-shaped changing part, the front body and the back body usually have the same dimensions, for fitting in the erect position, but when the foot is raised during walking or the leg is bent during sitting, fabric piles up on the popliteal spaces.

The crescent-shaped changing part described above will now be explained with reference to the accompanying drawings.

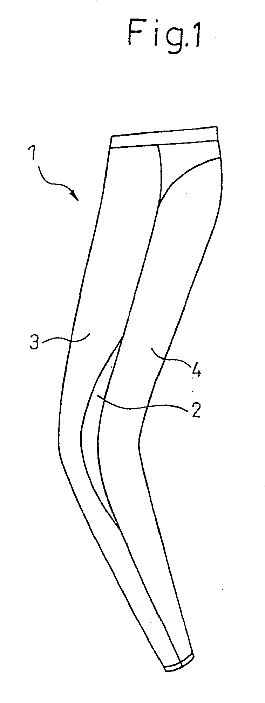

Fig. 1 is an outer side view of the left foot portion of sports tights as one embodiment of the medical or sports wear 1 of the invention. The crescent-shaped changing part 2 on the outer side section is located on the side section between the front body 3 and the back body 4.

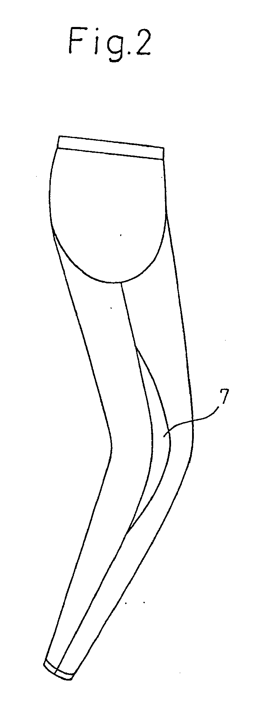

Fig. 2 is an inner side view of the left foot portion of sports tights as one embodiment of the medical or sports wear of the invention. The right foot section will not be explained. The crescent-shaped changing part 7 on the inner side section is located on the side section between the front body 3 and the back body 4, as in Fig. 1.

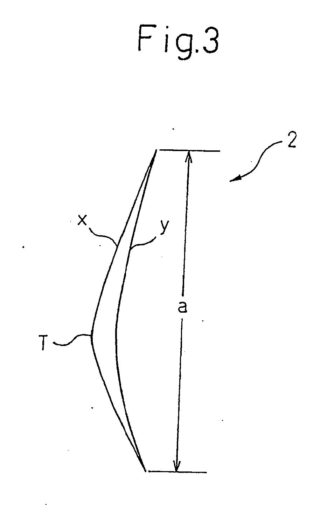

Fig. 3 shows the crescent-shaped changing part 2 on the outer side. In Fig. 3, the symbol x denotes the long arc close to a kneecap of the crescent-shaped changing part, the symbol y denotes the short arc close to a popliteal space of the crescent-shaped changing part, and the length (a) between the crossing sections of the two arcs of the crescent-shaped changing part is the length between the top edge and the bottom edge of the section where the two arcs of the crescent-shaped changing part 2 of the outer side section cross.

The lengths of the long arc x close to a kneecap and the short arc y close to a popliteal space are not particularly restricted so long as they are in ranges that create the desired dimensional difference in the medical or sports wear of the invention. However, while a large dimensional difference between the front and back bodies formed by the crescent-shaped changing part facilitates foot raising and is effective for large movement such as knee bending, an excessively large difference can produce wrinkles at the front of the knee in the erect position, and therefore the dimensional difference, i.e., the difference between the lengths of the long arc x close to a kneecap and the short arc y close to a popliteal space, is preferably 1 cm-8 cm and more preferably 2 cm-6 cm.

During wear, the top edge of the crescent-shaped changing part is located at least at a higher position than the top edge of the patella, but it is preferably at a position not significantly exceeding an area near the center of the thigh (the midpoint between the base of the hip and the center of the patella). This is because bending and extension of the hip joint has a greater effect on expansion of skin around the area near the hip joint.

The bottom edge of the crescent-shaped changing part is located at least at a lower position than the bottom edge of the patella, but it is preferably in a range not significantly exceeding the area near the calves (the largest region of the lower leg). This is because bending and extension of the ankle joint has a greater effect on expansion of skin around the area near the foot joint.

In Fig. 3, the length (a) between the crossing sections of the two arcs of the crescent-shaped changing part is not particularly restricted so long as it is within a range satisfying the conditions for the long arc x close to a kneecap, the short arc y close to a popliteal space and the top edge and bottom edge of the crescent-shaped changing part, and it may be 8-45 cm, for example.

The top part T of the crescent-shaped changing part is provided at a location such that the height of the top part T of the crescent-shaped changing part in the medical or sports wear of the invention during wear is essentially equal to the height of the top part of the kneecap of the wearer.

The preferred ranges for (a), x and y were explained above for the crescent-shaped changing part 2 on the outer side section, but the preferred ranges for (a), x and y for the crescent-shaped changing part 7 on the inner side section are also the same as for the crescent-shaped changing part 2 on the outer side section.

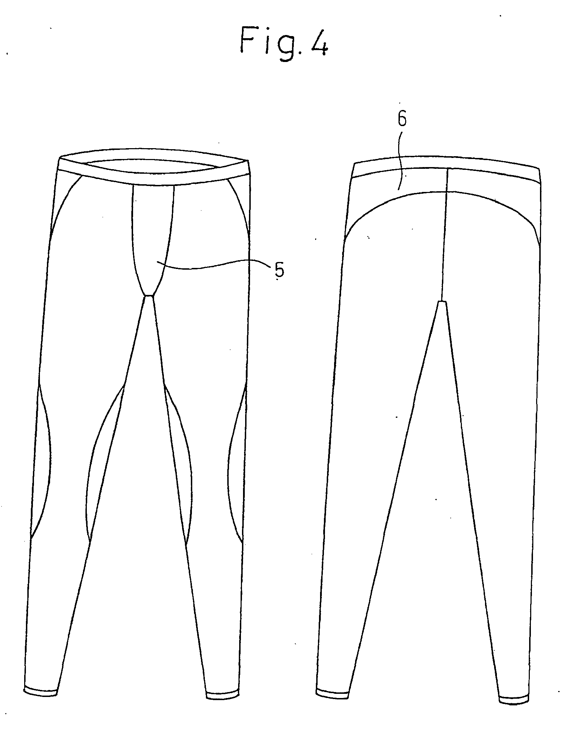

Fig. 4 is a front view and rear view of sports tights as one embodiment of the medical or sports wear of the invention. In Fig. 4, the numerals 5 and 6 denote the gusset section and flank section.

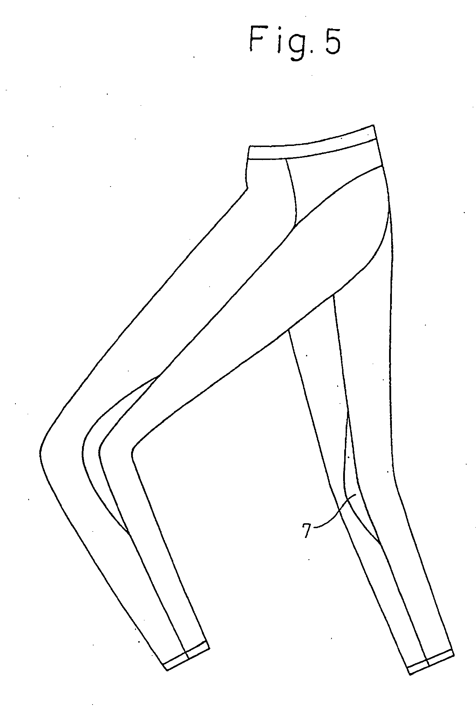

Fig. 5 is a left side view showing an image of walking with sports tights as one embodiment of the medical or sports wear of the invention. In Fig. 5, the numeral 7 denotes the crescent-shaped changing part on the inner side section.

The material of the crescent-shaped changing part is an elastic material and/or an air-permeable material which is the same as or different from the material of the sections other than the changing part. The "sections other than the changing part" may be, for example, the front body 3 and the back body 4. The "elastic material different from the material of the sections other than the changing part" may be, for example, a material with a greater expansion ratio that further increases the elasticity of the knee section, or it may be a material with a smaller expansion ratio that further decreases pile up at the popliteal space.

As used herein, the term "air permeability" refers to the degree of permeability for air. The "air-permeable material" used may be any material commonly used as an air-permeable material or a material with air permeability in the art, which does not cause perspiration during movement, and for example there may be used a material having an air permeability of 100 cm$^3$/cm$^2$·s or greater based on measurement according to JIS L1018 (measurement using a Frajour type tester). In order to effectively prevent perspiration during movement in summer season, the material may be a material having an air permeability of 300 cm$^3$/cm$^2$·s or greater, for example.

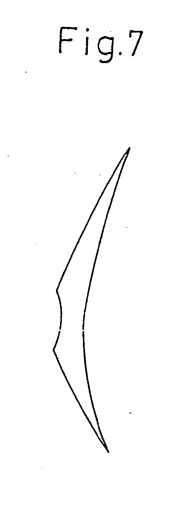

As used herein, "crescent-shaped changing part" includes not only shapes formed by two circular arcs, but also shapes in which the long arc x close to a kneecap and/or the short arc y close to a popliteal space are partially replaced by straight lines, and shapes in which the short arc y close to a popliteal space is a straight line. In addition, shapes in which the top part T of the crescent-shaped changing part is depressed to provide a separate knee pad (for example, the crescent-shaped changing part illustrated in Fig. 7) are also included by "crescent-shaped changing part" according to the invention. Such crescent-shaped changing part shapes may also be referred to as "curved sections".

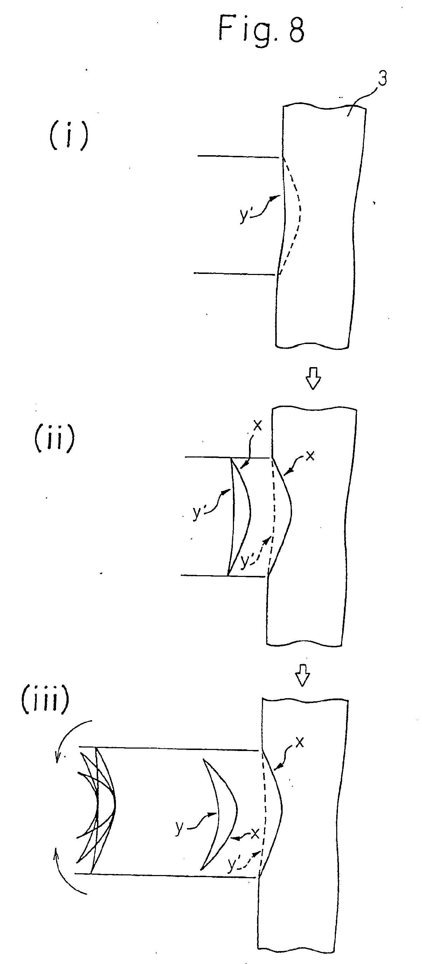

A process for determining the specific shape of the crescent-shaped changing part will now be explained with reference to Fig. 8. In Fig. 8(i), an imaginary line forming a crescent-shaped gusset is shown representing a fixed crescent-shaped changing part on the knee section of the front body 3. The short arc close to a popliteal space of the crescent-shaped gusset is denoted as y'. In (ii), the crescent-shaped gusset is cut out. The long arc close to a kneecap of the crescent-shaped gusset is denoted as x. Finally, in (iii), the crescent-shaped gusset is deformed while maintaining the length of the long arc x close to a kneecap. The short arc close to a popliteal space of the deformed crescent-shaped gusset is denoted as y. Greater deformation of the crescent-shaped gusset in the direction indicated by the arrow results in a shorter length of the short arc y close to a popliteal space, a greater dimensional difference and easier foot raising, but if the length of the short arc y close to a popliteal space is too short, wrinkles may occur in the knee front section in the erect position.

[Graded wear pressure]

As used herein, "leg" refers to the lower extremities, and it includes the femoral region, calves and ankles explained below.

Also, as used herein, the term "femoral region" refers to the region covering the adductor muscle group including the pectineal muscle, long adductor muscle, gracilis muscle and great adductor muscle, the quadriceps femoris muscles (femoral extensor group) including the sartorius muscle, rectus femoris muscle, intermediate great muscle, lateral great muscle and medial great muscle, and the femoral flexor group including the semimembraneous muscle, semitendinosus muscle and musculus biceps femoris.

As used herein, "calf" refers to the region covering the calf triceps muscle, the gastrocnemial muscle, the anterior tibial muscle and the extensor digitorum longus muscle.

As used herein, the term "ankle" refers to the area of shortest circumferential length between the calves and the malleolus.

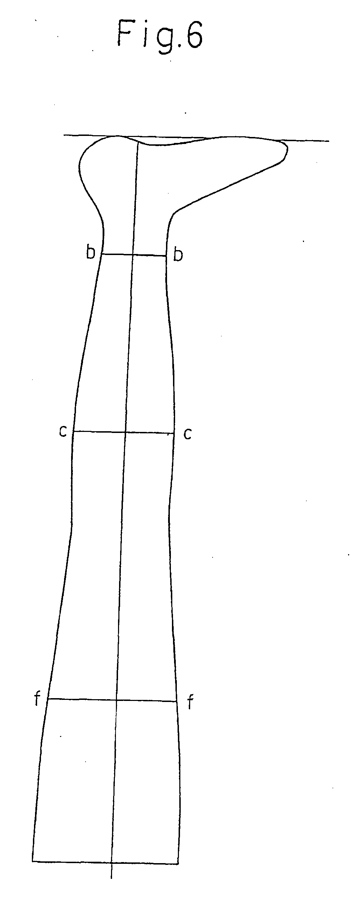

As used herein, the term "wear pressure" refers to pressure applied during wear. The wear pressure can be measured by fitting medical or sports wear of the invention onto a wooden wooden last shown in Fig. 6, for example, and using an MST-MK IV wear pressure meter by Salzmann for measurement.

The circumferential lengths of the wooden last shown in Fig. 6 are listed in Table 1 below. In Fig. 6, the letters b, c and f denote the ankle, calf and femoral regions, respectively.

Table 1. Circumferential lengths of wooden last shown in Fig. 6.

| Location | Circumferential length (cm) |
| --- | --- |
| b | 22.0 |
| c | 36.5 |
| f | 50.0 |

As used herein, "graded wear pressure" refers to different wear pressure at different locations within the leg region in the direction of body length. The graded wear pressure may be a variety of different types of graded wear pressure known in the field of pantyhose, and for example, it may be in the order: wear pressure on calf > wear pressure on femoral region, or: wear pressure on ankle > wear pressure on calf > wear pressure on femoral region.

In order to effectively aid the muscle pump effect on the calves of the legs and increase venous return that reduces swelling and circulatory disorders in the legs of the wearer, the preferred order is: wear pressure on ankle > wear pressure on calf > wear pressure on femoral region.

With the order: wear pressure on calf > wear pressure on femoral region, the wear pressure on the calf is preferably 7-24 hPa and more preferably 14-17.5 hPa, and the wear pressure on the femoral region is preferably 3-18 hPa and more preferably 8-16 hPa.

With the order: wear pressure on ankle > wear pressure on calf > wear pressure on femoral region, the wear pressure on the ankle is preferably 15 hPa-30 hPa and more preferably 18-27 hPa. The wear pressure on the calf and femoral region are the same as for the order: wear pressure on calf > wear pressure on femoral region.

[Sewn sections]

According to the invention, the sewn sections between the crescent-shaped changing part and the front body and back body are not particularly restricted and may be obtained by a variety of sewing methods, and for example, they may be obtained using a 4-needle flat seamer (top-and-bottom flat lock sewing machine), a 2-needle top-and-bottom flat lock sewing machine or a 3-needle top-and-bottom flat lock sewing machine. A flat seamer is particularly preferred. This will reduce the thickness of the changing part and avoid hindering of elongation of the material. The flat seamer may be used in "reverse" whereby sewing is performed while viewing the back side of the fabric, so that the sewing thread facing the skin does not stand out. This will further improve the feel on the skin.

According to the invention, a simple crescent-shaped changing part was used for three-dimensional design. Thus, the medical or sports wear of the invention has a low number of changing part, and as a result a lower number of sewn sections than conventional sport wear, so that the following advantages are obtained.

(1) Elongation of the material is not inhibited, and therefore the wear pressure does not easily vary even when the circumferential length at the femoral region or other regions varies during movement.
(2) Because elongation of the material is not inhibited, hindrance to exercise activity is minimal.
(3) The feel on the skin is soft, with no unpleasant feeling of contact.

The invention will now be explained in greater detail using examples and comparative examples, with the understanding that the invention is in no way limited in scope by the examples.

Examples

[Sports tights production example]

The sports tights shown in Fig. 1 and Fig. 4 were produced in the following manner.

The pattern papers for parts of sports tights comprising an outer side section crescent-shaped changing part 2, an inner side section crescent-shaped changing part 7, a front body 3, a back body 4, a gusset section 5 and a flank section 6 were positioned on the warp line of an elastic warp knit, and the parts (two parts) for the left and right outer side section crescent-shaped changing parts 2, two parts for the left and right inner side section crescent-shaped changing parts 7, two parts for the left and right front bodies 3, two parts for the left and right back bodies 4, a part for the gusset section 5 and two parts for the flank sections 6 were cut along a frame. The shapes of the parts for each outer side section crescent-shaped changing part 2 and inner side section crescent-shaped changing part 7 were symmetrical, and the difference in the lengths of the long arc x close to a kneecap and short arc y close to a popliteal space was 3 cm. The elongation percentage in the circumferential length direction of the elastic warp knit was 250%.

The parts for each outer side section crescent-shaped changing part 2 and the parts for each inner side section crescent-shaped changing part 7 were sewn with a flat seamer onto the two parts for the front bodies 3. Next, the parts for the flank sections 6 and the parts for the back bodies 4 were sewn onto the previously sewn two products using a flat seamer, in that order. The section below the hip was then sewn onto the two sewn products with a flat seamer, and the gusset section 5 was sewn on with a flat seamer. Waist rubber was then sewn onto the waist section by 2-needle top flat lock stitching. The fringes were folded in a 2-point zigzag to produce sports tights (1).

The sports tights (1) were fitted onto the wooden last shown in Fig. 6, and the wear pressures were measured. The wear pressures on the ankle, calf and femoral regions were 25 hPa, 17.5 hPa and 9 hPa, respectively.

[Example 1]

When the sports tights (1) were worn by several persons, the responses indicated that knee bending was facilitated during sports and a comfortable feeling of wear was obtained. The responses also indicated that fatigue during sports was reduced compared to conventional sports tights, and that recovery from fatigue after sports was more rapid.

[Example 2]

A monitor test was conducted to reconfirm the effect of the invention.
The conditions for the monitor test were as follows.

(1) Wear: Sports tights shown in Figs. 1 and 4, produced according to Production Example 1.
(2) Participants: 32 in total (23 men, 9 women).
(3) Period: Approximately 2 weeks.
(4) Activity:

(i) Sports (running, skiing, snowboard, etc.)
(Ii) Daily activities (walking, working, etc.)

(5) Evaluation:

(i) Ease of movement
(ii) Fatigue-reducing effect

A total of 6 different sports tights were prepared, of 3 types S, M and L each for the men and women, and the monitor test participants selected tights suited for their frame. All of the sports tights were designed so that the wear pressure on the ankles, calves and femoral regions were about 25 hPa, 17.5 hPa and 9 hPa, respectively.

[Ease of movement]

The monitor test participants evaluated ease of movement on the following 3-level scale.

Good (G): Same as without tights.
Poor (P): More difficult movement than without tights.

The results are summarized in Table 2 below.

Table 2. Monitor test results for ease of movement

| Ease of movement | G | P |
|---|---|---|
| Percentage of respondents | 95% | 5% |

As shown in Table 2, 95% of the monitor test participants responded that wearing the sports tights provided the same ease of movement as without tights. Considering that wearing conventional sports tights resulted in less ease of movement, it may be concluded that the effect of facilitating movement by wearing the sports tights of the invention was reconfirmed by the monitor test.
Furthermore, surprisingly, of the 95% of participants that responded that the ease of movement was the same as without tights, 56% responded that wearing the sports tights provided greater ease of movement than without them.

[Fatigue-reducing effect]

The monitor test participants evaluated the fatigue-reducing effect on the following 4-level scale. This was compared with the effect without wearing the sports tights.

Very good (VG): Very high fatigue-reducing effect.
Good (G): High fatigue-reducing effect.
Fair (F): Slight fatigue-reducing effect.

Poor (P): No fatigue-reducing effect.

The results are summarized in Table 3 below. One person who failed to respond was excluded from the calculation.

Table 3. Monitor test results for fatigue-reducing effect

| Fatigue-reducing effect | VG | G | F | P |
|---|---|---|---|---|
| Percentage of respondents | 23% | 71% | 6% | 0% |

As seen in Table 3, 23% of the participants responded that the sports tights had a very high fatigue-reducing effect compared to evaluation without the sports tights, while 71% of the participants responded that the sports tights had a higher fatigue-reducing effect than without the tights. Thus, 94% of the participants responded that the fatigue-reducing effect of the sports tights was high or very high. Thus, the fatigue-reducing effect of the sports tights of the invention was reconfirmed by the monitor test.

[Ease of positioning of crescent-shaped changing part]

The participants in the monitor test were asked to evaluate the ease of positioning of the crescent-shaped changing part of the sports tights to the prescribed position, i.e. the knee, on the following 5-level scale. Very Good (VG): Very easy positioning.

Good (G): Easy positioning.
Poor (P): Neither easy nor difficult positioning.
Bad (B): Difficult positioning.
Very Bad (VB): Very difficult positioning.

Table 4. Monitor test results for ease of positioning of crescent-shaped changing part

| Ease of positioning | VG | G | F | B | VB |
|---|---|---|---|---|---|
| Percentage of respondents | 31% | 50% | 13% | 6% | 0% |

As seen in Table 4, 31% of the participants responded that the sports tights were very easy to position, while 50% of the participants responded that the sports tights were easy to position. Thus, 81% of the participants responded that the sports tights were easy or very easy to position, and therefore the sports tights of the invention may be judged to be easy to position during wear.
The ease of positioning is believed to be due to the ease of positioning of the crescent-shaped changing part, which is externally easy to recognize, on the knee during wear.

Industrial Applicability

The medical or sports wear of the invention is wear which allows easy bending of the knee during sports, has minimal excess fabric on the popliteal space and has low unpleasant sense of pressure as well as a comfortable feeling during wear, and it is industrially useful wear that promotes venous return, reduces fatigue on the legs during sports and improves sports ability, and therefore promises to reduce fatigue and promote rapid recovery from fatigue after sports.

References Signs List

1    Medical or sports wear of the invention
2    Crescent-shaped changing part of outer side section
3    Front body
4    Back body
5    Gusset section
6    Flank section

7   Crescent-shaped changing part of inner side section
a   Length of crossing section of 2 arcs of crescent-shaped changing part
b   Ankle
c   Calf
f   Femoral region
x   Long arc close to kneecap
y   Short arc close to popliteal space
T   Top part of crescent-shaped changing part

**Claims**

1. A medical or sports wear (1) comprising an elastic material and having a front body (3) and a back body (4), wherein the medical or sports wear (1) has a crescent-shaped changing part (2) on the inner and outer sides of the knee, the crescent shaped changing part comprising a long arc (x) close to the kneecap and connected to the front body (3), and a short arc (y) close to the popliteal space and connected to the back body (4), so as to provide a dimensional difference between the front body (3) and the back body (4), and wherein graded wear pressure is applied to the leg of the user by the medical or sports wear (1) during wear.

2. The medical or sports wear (1) according to claim 1, wherein the difference between the length of the long arc (x) close to a kneecap and the length of the short arc (y) close to a popliteal space is 1 cm-8 cm.

3. The medical or sports wear (1) according to claim 1 or claim 2, wherein the material of the crescent-shaped changing part (2) is an elastic material which is the same as the material of the sections (3,4) other than the changing part (2).

4. The medical or sports wear according to any one of claims 1 to 3, wherein the elongation percentage in the circumferential length direction of the elastic material is greater than the elongation percentage in the height direction.

5. The medical or sports wear (1) according to any one of claims 1 to 4, wherein the elongation percentage in the circumferential length direction of the elastic material is in the range of 150%-500%.

6. The medical or sports wear (1) according to any one of claims 1 to 5, wherein the graded wear pressure is wear pressure in the following order: wear pressure on calf > wear pressure on femoral region, or wear pressure on ankle > wear pressure on calf > wear pressure on femoral region.

7. The medical or sports wear (1) according to any one of claims 1 to 6, wherein the wear pressure on the calf is 7-24 hPa, and the wear pressure on the femoral region is 3-18 hPa.

8. The medical or sports wear (1) according to any one of claims 1 to 7, wherein the crescent-shaped changing part (2) has a recess at the top part of the changing part (2).

9. The medical or sports wear (1) according to any one of claims 1 to 8, comprising a sewn section wherein the sewn section is a reverse flat seam.

10. The medical or sports wear (1) according to any one of claims 1 to 9, wherein the medical or sports wear (1) is selected from the group consisting of sports tights, leggins, swimming wear, tights, pantyhose and body suits.

11. The medical or sports wear (1) according to any one of claims 1 to 7, wherein the long arc (x) and the short arc (y) forming the crescent shaped changing part are two circular arcs.

12. The medical or sports wear (1) according to claim 11, wherein one or both circular arcs are partially replaced by a straight line.

**Patentansprüche**

1. Medizinische Kleidung oder Sportkleidung (1), das ein elastisches Material umfasst und ein Vorderteil (3) und ein Rückenteil (4) aufweist, wobei

die medizinische Kleidung oder Sportkleidung (1) ein bogenförmiges Wechselstück (2) an der inneren und äußeren Seite des Knies hat, wobei das bogenförmige Wechselstück einen langen Bogen (x) nahe der Kniescheibe und verbunden mit dem Vorderteil (3), und einen kurzen Bogen (y) nahe der Kniekehle und verbunden mit dem Rückenteil (4) umfasst, um einen dimensionalen Unterschied zwischen dem Vorderteil (3) und dem Rückenteil (4) bereitzustellen, und wobei ein abgestufter Tragedruck auf das Bein des Verwenders durch die medizinische Kleidung oder Sportkleidung (1) während des Tragens ausgeübt wird.

2.  Medizinische Kleidung oder Sportkleidung (1) nach Anspruch 1, wobei der Unterschied zwischen der Länge des langen Bogens (x) nahe eine Kniescheibe und der Länge des kurzen Bogens (y) nahe einer Kniekehle 1 cm-8 cm beträgt.

3.  Medizinische Kleidung oder Sportkleidung (1) nach Anspruch 1 oder Anspruch 2, wobei das Material des bogenförmigen Wechselstücks (2) ein elastisches Material ist, welches dasselbe Material ist wie das der Bereiche (3, 4), anders als das Wechselstück (2).

4.  Medizinische Kleidung oder Sportkleidung nach einem der Ansprüche 1 bis 3, wobei die Dehnung in Prozent in Richtung der Umfangslänge des elastischen Materials größer ist als die Dehnung in Richtung der Höhe.

5.  Medizinische Kleidung oder Sportkleidung (1) nach einem der Ansprüche 1 bis 4, wobei die Dehnung in Prozent in Richtung der Umfangslänge des elastischen Materials im Bereich von 150%-500% liegt.

6.  Medizinische Kleidung oder Sportkleidung (1) nach einem der Ansprüche 1 bis 5, wobei der abgestufte Tragedruck, ein Tragedruck in der folgenden Reihenfolge ist: Tragedruck auf Wade > Tragedruck auf Oberschenkelbereich oder Tragedruck auf Knöchel > Tragedruck auf Wade > Tragedruck auf Oberschenkelbereich.

7.  Medizinische Kleidung oder Sportkleidung (1) nach einem der Ansprüche 1 bis 6, wobei der Tragedruck auf der Wade 7-24hPa beträgt und der Tragedruck auf den Oberschenkelbereich 3-18hPa beträgt.

8.  Medizinische Kleidung oder Sportkleidung (1) nach einem der Ansprüche 1 bis 7, wobei das bogenförmige Wechselstück (2) eine Vertiefung am Oberteil des Wechselstücks (2) hat.

9.  Medizinische Kleidung oder Sportkleidung (1) nach einem der Ansprüche 1 bis 8, umfassend einen Nahtabschnitt, wobei der Nahtabschnitt eine rückseitige Flachnaht ist.

10. Medizinische Kleidung oder Sportkleidung (1) nach einem der Ansprüche 1 bis 9, wobei die medizinische Kleidung oder Sportkleidung (1) aus der Gruppe bestehend aus engen elastischen Trainingshosen (Sport-Tights), Leggins, Schwimmkleidung, Strümpfen, Strumpfhosen und Bodysuits ausgewählt ist.

11. Medizinische Kleidung oder Sportkleidung (1) nach einem der Ansprüche 1 bis 7, wobei der lange Bogen (x) und der kurze Bogen (y), die das bogenförmige Wechselstück bilden, zwei Rundbögen sind.

12. Medizinische Kleidung oder Sportkleidung (1) nach Anspruch 11, wobei ein Rundbogen oder beide Rundbögen teilweise durch eine gerade Linie ersetzt sind.

**Revendications**

1.  Vêtement à usage médical ou sportif (1) comprenant un matériau élastique et présentant un devant (3) et un dos (4), dans lequel

    - le vêtement à usage médical ou sportif (1) présente une partie permutable en forme de croissant (2) sur les côtés intérieur et extérieur du genou, la partie permutable en forme de croissant comprenant un arc allongé (x) près de la rotule et relié au devant (3), et un arc court (y) près de la région poplitée et relié au dos (4) de manière à offrir une différence de dimension entre le devant (3) et le dos (4), et dans lequel
    - une pression graduelle est appliquée sur la jambe de la personne qui le porte par le vêtement à usage médical ou sportif (1) pendant le port de celui-ci.

2.  Vêtement à usage médical ou sportif (1) selon la revendication 1, dans lequel la différence entre la longueur de l'arc allongé (x) près d'une rotule et la longueur de l'arc court (y) près d'une région poplitée s'élève entre 1 cm et 8 cm.

3. Vêtement à usage médical ou sportif (1) selon la revendication 1 ou la revendication 2, dans lequel le matériau de la partie permutable en forme de croissant (2) est un matériau élastique qui est le même que le matériau des sections (3, 4) autres que la partie permutable (2).

4. Vêtement à usage médical ou sportif (1) selon l'une quelconque des revendications 1 à 3, dans lequel le pourcentage de l'allongement dans le sens de la longueur sur la circonférence du matériau élastique est supérieur au pourcentage de l'allongement dans le sens de la hauteur.

5. Vêtement à usage médical ou sportif (1) selon l'une quelconque des revendications 1 à 4, dans lequel le pourcentage d'allongement dans le sens de la longueur sur la circonférence du matériau élastique est compris dans la plage de 150 % à 500 %.

6. Vêtement à usage médical ou sportif (1) selon l'une quelconque des revendications 1 à 5, dans lequel la pression graduelle résultant du port du vêtement est une pression résultant du port du vêtement dans l'ordre suivant : pression résultant du port du vêtement sur le mollet > pression résultant du port du vêtement sur la région fémorale, ou pression résultant du port du vêtement sur la cheville > pression résultant du port du vêtement sur le mollet > pression résultant du port du vêtement sur la région fémorale.

7. Vêtement à usage médical ou sportif (1) selon l'une quelconque des revendications 1 à 6, dans lequel la pression résultant du port du vêtement sur le mollet s'élève à 7 à 24 hPa, et la pression résultant du port du vêtement dans la région fémorale s'élève à 3 à 18 hPa.

8. Vêtement à usage médical ou sportif (1) selon l'une quelconque des revendications 1 à 7, dans lequel la partie permutable en forme de croissant (2) présente un évidement dans la partie supérieure de la partie permutable (2).

9. Vêtement à usage médical ou sportif (1) selon l'une quelconque des revendications 1 à 8, comprenant une section cousue dans laquelle la section cousue est une couture plaque arrière.

10. Vêtement à usage médical ou sportif (1) selon l'une quelconque des revendications 1 à 9, dans lequel le vêtement ou article de sport à usage médical ou sportif (1) est choisi dans le groupe consistant en collants de sport, leggins, tenue de natation, collants, bas et maillots.

11. Vêtement à usage médical ou sportif (1) selon l'une quelconque des revendications 1 à 7, dans lequel l'arc allongé (x) et l'arc court (y) formant la partie permutable en forme de croissant sont deux arcs circulaires.

12. Vêtement à usage médical ou sportif (1) selon la revendication 11, dans lequel un arc ou les deux arcs circulaire(s) est/sont partiellement remplacé(s) par une ligne droite.

# Fig.1

# Fig.2

# Fig.3

# Fig.4

# Fig. 5

# Fig.6

# Fig.7

# Fig. 8

(i)

(ii)

(iii)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000290806 A
- JP 3279403 A
- JP 10130915 A
- JP 2008513623 A
- JP 10110306 A